(19) Europäisches Patentamt
European Patent Office
Office européen des brevets

(11) **EP 3 889 808 A1**

(12) **EUROPEAN PATENT APPLICATION**

(43) Date of publication:
06.10.2021 Bulletin 2021/40

(51) Int Cl.:
*G06F 17/18* (2006.01)   *A61B 5/377* (2021.01)

(21) Application number: 21160027.5

(22) Date of filing: 01.03.2021

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(30) Priority: 23.03.2020 JP 2020051649

(71) Applicant: Ricoh Company, Ltd.
Tokyo 143-8555 (JP)

(72) Inventors:
• Misaka, Yoshihiro
Ohta-ku, Tokyo 143-8555 (JP)
• Morise, Hirofumi
Ohta-ku, Tokyo 143-8555 (JP)
• Kudo, Kiwamu
Ohta-ku, Tokyo 143-8555 (JP)

(74) Representative: White, Duncan Rohan
Marks & Clerk LLP
Fletcher House
Heatley Road
The Oxford Science Park
Oxford OX4 4GE (GB)

(54) **INFORMATION ANALYSIS APPARATUS**

(57) An information analysis apparatus (1) includes a feature quantity calculation unit (12) and an output unit (13). The feature quantity calculation unit (12) is configured to calculate a feature quantity on a biological body of a single subject or a group of a plurality of subjects, from data groups acquired under N acquisition conditions (k) where N is equal to or larger than three. The output unit (13) is configured to output the feature quantity. The feature quantity is represented by Expression (1) below using numbers of acquisitions ($n_k$), average values ($<X_k>$), unbiased variances ($S_k^2$), and contribution ratios ($w_k$) under the acquisition conditions (k).

$$t = \frac{w_1\langle X_1\rangle + w_2\langle X_2\rangle + w_3\langle X_3\rangle + \cdots + w_N\langle X_N\rangle}{\sqrt{w_1^2 \times \frac{S_1^2}{n_1} + w_2^2 \times \frac{S_2^2}{n_2} + w_3^2 \times \frac{S_3^2}{n_3} + \cdots + w_N^2 \times \frac{S_N^2}{n_N}}} \qquad (1)$$

(1)

FIG.1

**Description**

BACKGROUND OF THE INVENTION

1. Field of the Invention

The present invention relates to an information analysis apparatus.

2. Description of the Related Art

As an apparatus that analyzes biological information on a subject and extracts biological feature quantities of the subject or a group to which the subject belongs, an information analysis apparatus that uses data measured by a biological function measurement apparatus, such as magnetoencephalography (MEG), electroencephalography (EEG), functional magnetic resonance imaging (fMRI), positron emission tomography (PET), near-infrared spectroscopy (NIRS), or an optically pumped atomic magnetometer (OPM), has been known.

The information analysis apparatus as described above is able to examine brain functions of a human being by providing, for example, sound stimulation or visual stimulation to the subject and measuring brain neural activity that is induced in a sensory area or the like or measuring corresponding secondary activity.

However, in general, the brain neural activity is usually a faint signal, and measured data contains, in a superimposed manner, a signal that is derived from an electrical apparatus, such as a stimulator, or derived from heartbeats or electrical activities of nerves and muscles of a subject, in addition to a signal that is derived from the intended brain neural activity, and the superimposed signal becomes noise when a feature of the target brain neural activity is to be extracted. Therefore, for example, a technique of separating noise from the signal derived from the brain activity by using a technique of a frequency response filter or the like has been known.

A magnetic field generated with the brain neural activity is weak, and MEG includes noise that is derived from an electrical apparatus, such as a stimulator, or derived from heartbeats or electrical activities of nerves and muscles of a subject, in addition to including the brain neural activity. Therefore, a technique of separating noise from the signal derived from the brain activity by using a technique of a frequency response filter or the like has been known.

However, in the technique of separating noise from the signal derived from the brain activity in the conventional technique as described above, it is difficult to fully eliminate certain noise, such as random noise of a sensor itself, which is in the same frequency band as that of a biological signal.

The noise is not fully eliminated as described above leads a problem when, in particular, a feature quantity of a subject or a group is to be extracted from biological response data that are acquired under a plurality of conditions. For example, when brain activity related to sensory integration of vision and hearing is to be extracted from a group A of brain activity data that is acquired a plurality of number of times under a condition A that visual stimulation is provided to a subject, from a group B of brain activity data that is acquired a plurality of number of times under a condition B that auditory stimulation is provided, and from a group C of data that is acquired a plurality of number of times under a condition C that visual stimulation and auditory stimulation are simultaneously provided to the subject, and if a simple sum of an average value of the data group A and an average value of the data group B is compared with an average value of the group C, sensory integrative activity may be underestimated by a common factor component that is caused by noise. Therefore, in a situation in which noise is not fully eliminated, detection accuracy of feature quantities (for example, a biomarker) based on comparison of biological activities caused by, for example, various types of stimulation is degraded, which is a problem.

The related techniques are described in Japanese Unexamined Patent Application Publication No. 2015-100, Oliver Werner Sakowitz et.al., "Bisensory stimulation increases gamma-responses over multiple cortical regions", Cognitive Brain Research, Volume 11, Issue 2, April 2001, Pages 267-279, etc.

The present invention has been made in view of the foregoing situation, and an object of the present invention is to improve detection accuracy of feature data obtained by comparison of information.

SUMMARY OF THE INVENTION

An information analysis apparatus includes a feature quantity calculation unit and an output unit. The feature quantity calculation unit is configured to calculate a feature quantity on a biological body of a single subject or a group of a plurality of subjects, from data groups acquired under N acquisition conditions (k) where N is equal to or larger than three. The output unit is configured to output the feature quantity. The feature quantity is represented by Expression (1) below using numbers of acquisitions ($n_k$), average values ($<X_k>$), unbiased variances ($S_k^2$), and contribution ratios ($w_k$) under the acquisition conditions (k).

$$t = \frac{w_1\langle X_1\rangle + w_2\langle X_2\rangle + w_3\langle X_3\rangle + \cdots + w_N\langle X_N\rangle}{\sqrt{w_1^2 \times \dfrac{S_1^2}{n_1} + w_2^2 \times \dfrac{S_2^2}{n_2} + w_3^2 \times \dfrac{S_3^2}{n_3} + \cdots + w_N^2 \times \dfrac{S_N^2}{n_N}}} \qquad (1)$$

According to an aspect of the present invention, it is possible to improve detection accuracy of feature quantity by comparison of information.

BRIEF DESCRIPTION OF THE DRAWINGS

FIG. 1 is a block diagram illustrating a functional configuration example of an information analysis apparatus according to an embodiment;
FIG. 2 is a flowchart illustrating an example of an operation performed by the information analysis apparatus according to the embodiment;
FIG. 3 is an explanatory diagram for explaining a specific example of a feature quantity calculation;
FIG. 4 is an explanatory diagram for explaining a comparative example;
FIG. 5 is an explanatory diagram for explaining a specific example of the feature quantity calculation;
FIG. 6 is an explanatory diagram for explaining another specific example of the feature quantity calculation;
FIG. 7 is an explanatory diagram for explaining still another specific example of the feature quantity calculation;
FIG. 8 is an explanatory diagram for explaining a display example; and
FIG. 9 is an explanatory diagram for explaining another display example.

The accompanying drawings are intended to depict exemplary embodiments of the present invention and should not be interpreted to limit the scope thereof. Identical or similar reference numerals designate identical or similar components throughout the various drawings.

DESCRIPTION OF THE EMBODIMENTS

The terminology used herein is for the purpose of describing particular embodiments only and is not intended to be limiting of the present invention.
As used herein, the singular forms "a", "an" and "the" are intended to include the plural forms as well, unless the context clearly indicates otherwise.
In describing preferred embodiments illustrated in the drawings, specific terminology may be employed for the sake of clarity. However, the disclosure of this patent specification is not intended to be limited to the specific terminology so selected, and it is to be understood that each specific element includes all technical equivalents that have the same function, operate in a similar manner, and achieve a similar result.
An embodiment of the present invention will be described in detail below with reference to the drawings.
Embodiments of an information analysis apparatus and an information analysis method will be described in detail below with reference to the accompanying drawings.

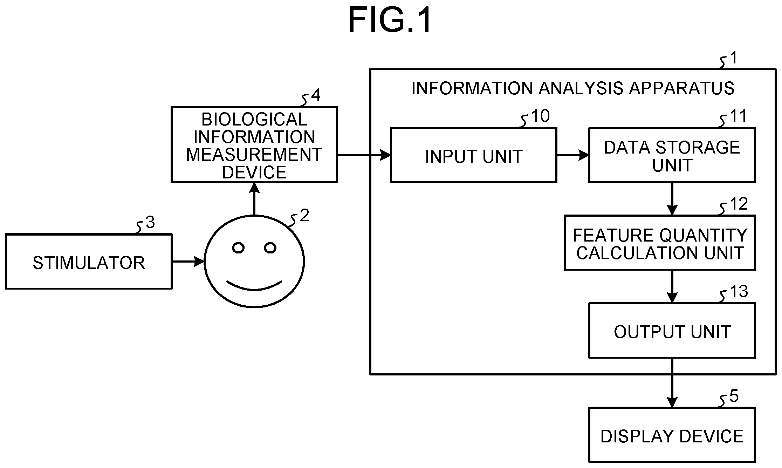

FIG. 1 is a block diagram illustrating a functional configuration example of an information analysis apparatus 1 according to an embodiment. As illustrated in FIG. 1, the information analysis apparatus 1 is an apparatus that repeatedly measures, by a biological information measurement device 4, biological information on a subject 2 to whom various types of stimulation are provided by a stimulator 3, and analyzes the repeatedly measured biological information (data group).
For example, the information analysis apparatus 1 is able to adopt data of magnetoencephalography (MEG) or the like, in which sound stimulation and visual stimulation are provided to the subject 2 and a biomagnetic field generated by brain neural activity that is induced in the sensory area or the like is repeatedly measured and analyzed.
The stimulator 3 provides various types of stimulation to the subject 2 at predetermined intensity. Examples of types of stimulation provided to the subject 2 by the stimulator 3 include stimulation to sight, hearing, taste, smell, or somatic sense (touch, pain, and the like), electrical stimulation that induces brain activity for exercise, language, memory, attention, performance, sociality, emotion, and the like, physical stimulation, such as magnetic stimulation and ultrasound stimulation, training, rehabilitation, and medication. Details (a type, strength, and the like) of stimulation that is provided to the subject 2 by the stimulator 3 is input, as input information on stimulation, to the information analysis apparatus 1.
The biological information measurement device 4 repeatedly measures information on a potential, a magnetic field, and the like that are generated by a pulse, blood pressure, a respiratory rate, or neuroelectronic activity of the subject 2. Meanwhile, the repeatedly measured biological information may be a statistic (an average value, a maximum value, a minimum value, a median value, dispersion, or the like) for a plurality of time points in a predetermined time period. The biological information measurement device 4 inputs biological information that is continuously or intermittently repeatedly measured to the information analysis apparatus 1 together with a measurement condition (a measurement time point, attributes of the subject 2, input information on the stimulation, or the like).

The attributes of the subject 2 include various types of attributes, such as identification information (ID) for identifying the subject 2, physical description, such as gender and a body height, of the subject 2, presence or absence of a disease, a disease type, a genotype, and the like. The attributes of the subject 2 as described above may be input in advance when the biological information measurement device 4 performs measurement or may be read from a memory or the like, for example.

In the present embodiment, magnetic field data that is repeatedly measured by the biological information measurement device 4 is input to the information analysis apparatus 1. Meanwhile, the biological information (repeatedly measured data group) or the like to be analyzed by the information analysis apparatus 1 is not limited to the magnetic field. For example, it may be possible to use data that is measured by EEG, fMRI, PET, NIRS, or an OPM as a general biological function measurement apparatus.

Alternatively, the biological information analysis apparatus 1 may analyze a pulse, blood pressure, a respiratory rate, cerebral blood flow, eye motion, body motion, or the like of the subject 2, which is repeatedly measured when various types of stimulation is provided to the subject 2.

The information analysis apparatus 1 includes an input unit 10, a data storage unit 11, a feature quantity calculation unit 12, and an output unit 13. Specifically, the information analysis apparatus 1 functions as the input unit 10, the data storage unit 11, the feature quantity calculation unit 12, and the output unit 13 by causing a processor, such as a central processing unit (CPU), to read a program and execute the program (details thereof will be described later).

The input unit 10 is a processing unit that receives input of various types of data. Specifically, the input unit 10 receives biological information that is repeatedly measured by the biological information measurement device 4, and the measurement condition (the measurement time point, the attributes of the subject 2, the input information on the stimulation).

Further, the input unit 10 receives, by user input, various types of setting, such as a calculation condition on a calculation of a feature quantity and an output condition for a calculation result. The calculation condition includes, for example, designation of a data group to be compared, setting of a parameter (a contribution ratio or the like) related to the calculation, and the like. Furthermore, the output condition includes, for example, designation of a graph to be displayed.

The data storage unit 11 is a database or the like for storing various types of data that are input to and received by the input unit 10. Specifically, the data storage unit 11 stores therein the biological information (data group) that is repeatedly measured by the biological information measurement device 4 and the measurement condition (the measurement time point, the attributes of the subject 2, and the input information on the stimulation) in an associated manner. The association of the data as described above makes it possible to perform comparison for each of the attributes of the subject 2 and each type of stimulation with respect to the biological information (data group) stored in the data storage unit 11.

The feature quantity calculation unit 12 is a processing unit that compares the biological information (data group) on the subject 2 that is repeatedly measured by the biological information measurement device 4, and calculates the feature quantity (details will be described later). The output unit 13 is a processing unit that outputs and displays the feature quantity calculated by the feature quantity calculation unit 12 onto a display device 5 in accordance with the set output condition.

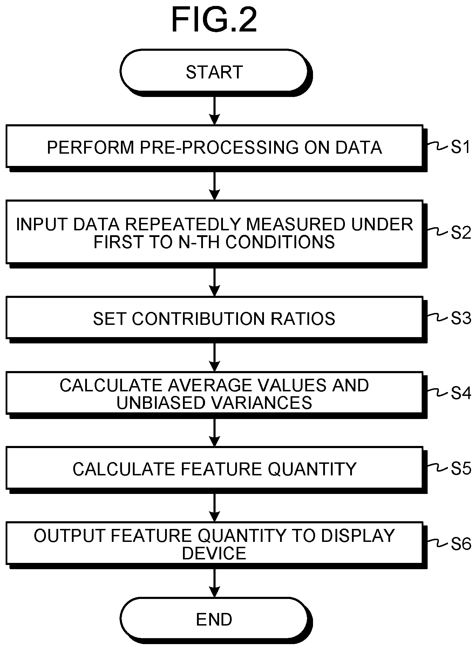

Operation of the information analysis apparatus 1 and a process of calculating the feature quantity will be described in detail below. FIG. 2 is a flowchart illustrating an example of the operation performed by the information analysis apparatus 1 according to the embodiment.

As illustrated in FIG. 2, if the process is started, for example, the input unit 10 performs pre-processing on biological information (data groups of biological signals), which is repeatedly measured under a plurality of conditions (for example, types of stimulation) by the biological information measurement device 4. Meanwhile, the pre-processing may be performed on the biological information measurement device 4 side.

Specifically, with respect to the biological information (the data groups of the biological signals) that is repeatedly measured under the plurality of conditions (for example, the types of stimulation) by the biological information measurement device 4, noise in a certain frequency band that is apparently different from frequency bands of the biological signals is eliminated by using a frequency response filter or the like (a bandpass filter, a low-pass filter, a high-pass filter, a notch filter, or the like). Further, to focus on the information on brain activity in the biological information, data from which information other than the brain activity, e.g., heartbeats, electrical activity of nerves and muscles, blinks, and the like that are included in the biological information, is excluded by using principal component analysis (PCA) or independent component analysis (ICA) may be adopted.

Subsequently, the input unit 10 receives input of data (the data groups of the biological signals) that is subjected to the pre-processing and that is repeatedly measured under a first to an N-th conditions (however, N≥3) (S2), and stores the input data together with the conditions in the data storage unit 11. The conditions include various conditions, such as a condition for each type of stimulation or a condition for each of the attributes of the subject 2.

Subsequently, the feature quantity calculation unit 12 reads the data (the data groups of the biological signals) that is repeatedly measured under the first to the N-th conditions from the data storage unit 11, compares the data groups, and calculates feature quantity (for example, a biomarker) (S3 to S5).

Specifically, the feature quantity calculation unit 12 sets a contribution ratio ($w_k$) with respect to the feature quantity of each of the data groups (S3). As for the setting of the contribution ratio ($w_k$), a user may set arbitrary contribution ratios or it may be possible to use a set of previously defined contribution ratios that are stored in advance in a memory or the like.

It is preferable that at least one of the contribution ratios is positive and at least another one of the contribution ratios is negative. For example, if $w_A>0$, $w_B>0$, and $w_C<0$, it is possible to eliminate influence that is included in the data group C and that is common to the data group A or the group B, so that it is possible to improve accuracy of the calculation of the feature quantity.

For example, it is assumed that data groups A, B, and C are obtained under respective conditions a, b, and c. However, the condition c includes a factor that is common to both of the condition a and the condition b.

A situation in which $w_i=-w_j$ indicates that the contribution ratios have opposite signs and the same magnitude between the conditions having a common factor. Therefore, two expressions are obtained such that $w_a=-1$ and $w_c=1$ with respect to the contribution ratios under the condition a and the condition b, and such that $w_b=-1$ and $w_c=1$ with respect to the contribution ratios under the condition b and the condition c.

Further, a numerator of the feature quantity (t) is represented as follows.

$$t=(w_a/X_a+w_b/X_b+w_c/X_c)$$

$$=(X_c-(X_a+X_b))$$

In the numerator part, the magnitudes of $X_a$ and $X_b$ are multiplied by one, and a difference from a multiple of the magnitude of $X_c$ is obtained. Therefore, in the obtained feature quantity, a feature quantity that is different from a simple linear sum of a+b, i.e., a non-linear effect, is extracted.

Furthermore, it is preferable that the contribution ratio meets Expression (1) below.

$$\left|\sum_i^N w_i\right| \Big/ \sum_i^N |w_i| \geqq 1/3 \qquad\qquad (1)$$

Next, a condition that needs to be met by the contribution ratios will be described using a typical example. Contribution ratios in conditions $a_1$, $a_2$, ..., $a_N$ are respectively denoted by $W_1$, $W_2$, ..., $W_N$. However, it is assumed that the conditions $a_1$ ..., $a_{N-1}$ do not include a clearly common factor, and the condition $a_N$ includes a common factor to each of the conditions $a_1$, ..., $a_{N-1}$. In this case, if the contribution ratios of the conditions $a_1$, ..., $a_{N-1}$ are set so as to have the same signs and the same magnitudes and only the contribution ratio of the condition $a_N$ is set so as to have the same magnitude and an opposite sign, i.e., such that $W_1=W_2=...=W_{N-1}=-W_N$, the feature quantity t has an effect to eliminate factors that are uniquely included in the conditions $a_1$, ..., $a_{N-1}$, but a factor (noise factor) that is commonly included in the conditions $a_1$, ..., $a_{N-1}$ is excessively counted. In the typical example of the present embodiment as described above, a left side of Expression (1) is represented as (N-2)/N, which becomes 1/3 or larger if N≥3. Therefore, it is preferable to meet Expression (1) because it is possible to improve accuracy of the feature quantity by using a method of the present invention.

Subsequently, the feature quantity calculation unit 12 calculates an average value and an unbiased variance with respect to the number of repeated measurements in each of the data groups A, B, and C (S4).

In general, if the number of repeated measurements n ≥ 30, it is possible to assume that approximation is possible such that a data distribution follows a normal distribution. Therefore, because power of test of a difference in the average value increases as the distribution approaches the normal distribution, it is preferable to increase the number of repeated measurements.

Subsequently, the feature quantity calculation unit 12 calculates the feature quantity by using the number of repeated measurements in each of the data groups A, B, and C and by using the average value and the unbiased variance with respect to the number of repeated measurements (S5). Then, the output unit 13 outputs and displays the feature quantity calculated by the feature quantity calculation unit 12 onto the display device 5 (S6).

Specifically, the feature quantity calculation unit 12 calculates the feature quantity (t) by assigning, to Expression (2) below, the number of repeated measurements in the N-th data group as $n_k$, the average value of the N-th data group as $<X_k>$, the unbiased variance of the N-th data group as $S_k^2$, and the contribution ratio of the feature quantity of the N-th data group as $W_k$. Meanwhile, if n is fully large, the unbiased variance and a sampling variance have approximately the same values, so that it may be possible to use the sampling variance instead of the unbiased variance.

$$t = \frac{w_1\langle X_1\rangle + w_2\langle X_2\rangle + w_3\langle X_3\rangle + \cdots + w_N\langle X_N\rangle}{\sqrt{w_1^2 \times \frac{S_1^2}{n_1} + w_2^2 \times \frac{S_2^2}{n_2} + w_3^2 \times \frac{S_3^2}{n_3} + \cdots + w_N^2 \times \frac{S_N^2}{n_N}}} \qquad (2)$$

Here, complementary explanation of a theoretical background of Expression (2) above for calculating the feature quantity will be described below. A test statistic (feature quantity) t is derived between a single population A (the data group A) and a sum (B+C) of two populations (the data groups B and C). When $n_A$ samples, $n_B$ samples, and $n_C$ samples are extracted from the populations for which population distributions follow respective normal distributions N ($\mu_A$, $\sigma^2_A$), N ($\mu_B$, $\sigma^2_B$), and N ($\mu_C$, $\sigma^2_C$), sample means $<X_A>$, $<X_B>$, and $<X_C>$ of samples that are extracted from the populations respectively follow $<X_A>\sim$N ($\mu_A$, $\sigma^2_A$), $<X_B>\sim$N ($\mu_B$, $\sigma^2_B$), and $<X_C>\sim$N ($\mu_C$, $\sigma^2_C$).

Here, a single data is extracted from each of the sample B and the sample C, and a distribution of a sum of the extracted data will be examined. If random variables $<X_B>$ and $<X_C>$ are independent of each other, the sum of the sample means ($<X_B>+<X_C>$) follows a distribution represented by Expression (3) below due to the additivity of normal distribution.

$$(\langle X_B\rangle + \langle X_C\rangle)\sim N\left((\mu_B + \mu_C), (\sigma_B^2 + \sigma_C^2)\right) \qquad (3)$$

Similarly, if a random variable $<X_A>$ and the random variable ($<X_B>+<X_C>$) are independent of each other, a difference between the random variables follow a distribution represented by Expression (4) below.

$$\langle X_A\rangle - (\langle X_B\rangle + \langle X_C\rangle)\sim N\left(\mu_A - (\mu_B + \mu_C), \left(\sigma_A^2 + (\sigma_B^2 + \sigma_C^2)\right)\right) \qquad (4)$$

In general, in X~N($\mu$, $\sigma$), it is possible to perform standardization such that Z=(X-$\mu$)/$\sigma$~N(0, 1). Therefore, Expression (5) below is obtained by performing standardization on Expression above.

$$Z = \frac{(\langle X_A\rangle - (\langle X_B\rangle + \langle X_C\rangle)) - (\mu_A - (\mu_B + \mu_C))}{\sqrt{\sigma_A^2 + (\sigma_B^2 + \sigma_C^2)}}\sim N(0,1) \qquad (5)$$

Here, $\sigma^2_A$, $\sigma^2_B$, and $\sigma^2_C$ are variances of populations and unknown. Therefore, a variance of a sample that can be calculated from the sample is used as a substitute. In general, the variance $\sigma^2$ of the population is estimated such that $\sigma^2=S^2/n$ by using an unbiased variance $S^2$ of the sample and the number n of samples. Therefore, by assignment to Expression (5) above, Expression (6) below is obtained.

$$t = \frac{(\langle X_A\rangle - (\langle X_B\rangle + \langle X_C\rangle)) - (\mu_A - (\mu_B + \mu_C))}{\sqrt{\left(\frac{S_A^2}{n_A} + \frac{S_B^2}{n_B} + \frac{S_C^2}{n_C}\right)}}\sim N(0,1) \qquad (6)$$

A null hypothesis is the assumption that there is no difference between an average value of the population A and an average value of the population (B+C), so that $\mu_A=(\mu_B+\mu_C)$ and the feature quantity (t) as represented by Expression (7) below is obtained.

$$t = \frac{\langle X_A \rangle - (\langle X_B \rangle + \langle X_C \rangle)}{\sqrt{\left(\frac{s_A^2}{n_A} + \frac{s_B^2}{n_B} + \frac{s_C^2}{n_C}\right)}} \sim N(0,1) \tag{7}$$

Here, explanation on the three data groups A, B, and C has been described, but even if the number of data groups increases to N, it is possible to adopt the same method in an extended manner.

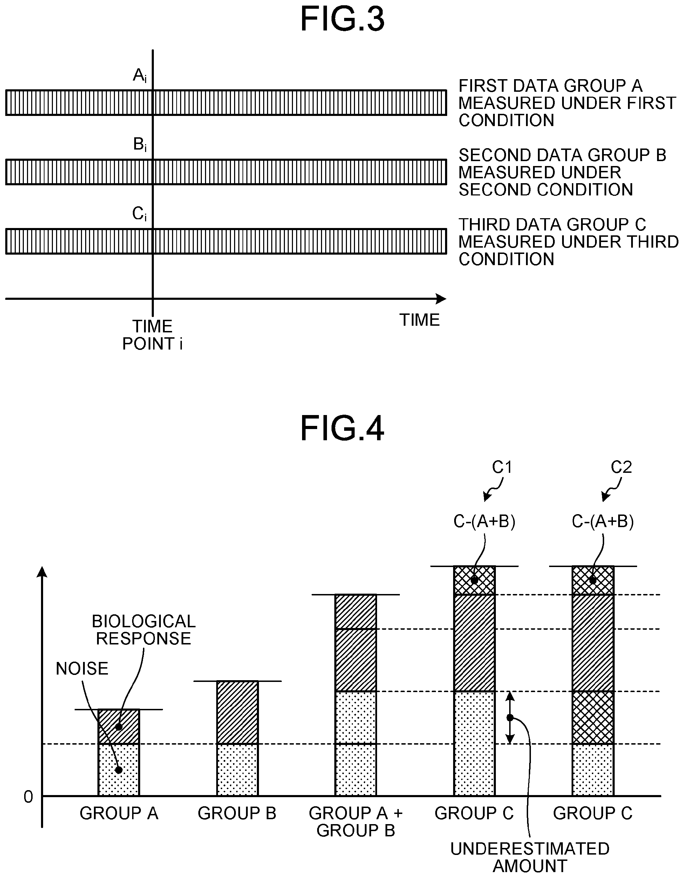

FIG. 3 is an explanatory diagram for explaining a specific example of a feature quantity calculation. As illustrated in FIG. 3, the feature quantity (t) at a time point i is calculated with respect to a first data group A that is measured under a first condition, a second data group B that is measured under a second condition, and a third data group C that is measured under a third condition.

Meanwhile, for simplicity of explanation, the data group A, the data group B, and the data group C that includes a factor common to both of the data group A and the data group B are used. Therefore, an expression for calculating the feature quantity is represented as Expression (8) below.

$$t = \frac{w_1\langle X_1 \rangle + w_2\langle X_2 \rangle + w_3\langle X_3 \rangle + \cdots + w_N\langle X_N \rangle}{\sqrt{w_1^2 \frac{s_1^2}{n_1} + w_2^2 \frac{s_2^2}{n_2} + w_3^2 \frac{s_3^2}{n_3} + \cdots + w_N^2 \frac{s_N^2}{n_N}}} \rightarrow$$

$$t = \frac{\langle X_C \rangle - (\langle X_A \rangle + \langle X_B \rangle)}{\sqrt{\left(\frac{s_A^2}{n_A} + \frac{s_B^2}{n_B} + \frac{s_C^2}{n_C}\right)}} \tag{8}$$

First, the feature quantity calculation unit 12 sets contribution ratios such that $w_{Ci}=1$ and $w_{Ai}=w_{Bi}=-1$ at the time point i. Subsequently, the feature quantity calculation unit 12 calculates, for each of measurement conditions, an average value and an unbiased variance of the repeatedly measured data at the time point i. Then, the feature quantity calculation unit 12 assigns the number of repeated measurements for each of the measurement conditions and the average value and the unbiased variance of the number of repeated measurements to Expression (8) above to calculate the feature quantity.

Here, the example has been explained in which the feature quantity at the time point i is calculated under all of the conditions for the data group A, the data group B, and the data group C; however, it is possible to calculate the feature quantity by the same procedure at each of time points i, j, and k ($i \neq j \neq k$) for the respective conditions.

FIG. 4 is an explanatory diagram for explaining a comparative example, and in particular, illustrates a comparative example with respect a case C1 based on a conventional technique and a case C2 based on the present embodiment.

As illustrated in FIG. 4, in the case C1 based on the conventional technique, if a simple sum of a measured value of a group A and a measured value of a group B is compared with an average value of a group C, a common factor part (underestimated amount) due to noise is underestimated.

In general, an average value of noise is stochastically converged to a constant by repeating measurements. Here, by performing correction such that the average value of noise reaches zero, an average value of measurement values and an average value of biological signals become equal to each other, and the average value of the biological signals does not increase and decrease due to noise. In the present embodiment, the feature quantity calculation unit 12 calculates, by using Expression (2) above, the feature quantity by comparing the average values of the biological signals that are measured under a plurality of conditions. Therefore, in the information analysis apparatus 1, as indicated by the case C2, when the feature quantity of the biological signals obtained under at least three or more measurement conditions is to be extracted, it is possible to reduce an influence of noise that can hardly be eliminated by conventional noise elimination, and it is possible to improve accuracy of a biomarker that is based on the biological signals.

Furthermore, the magnitude of noise that is included in each of measurement values that are measured under different conditions is not always a constant value among the conditions. In the present embodiment, even if a variance of noise included in the repeatedly measured measurement value is different among the conditions, because standardization is performed in the denominator term of the expression for calculating the feature quantity, it is possible to perform comparison with other conditions without the influence of the noise.

For example, when a biological response is compared between time points at which noise variances are different, or when a biological response is compared among the plurality of subjects 2 with different noise variances, it is possible to perform comparison without regard to a difference in the magnitude of the noise.

Furthermore, in general, even if measurements are performed 100 times, in reality, some data are not usable for analysis due to an artifact or the like caused by body motion of the subject 2. It is often the case that data usable for analysis are obtained such that $n_a$=78 under the condition a, $n_b$=65 under the condition b, and $n_c$=82 under the condition c.

Here, the condition c is set so as to include a factor that is common to the condition a and the condition b. In this case, the number of measurements is different between the condition a and the condition b, and therefore, when a simple sum of the condition a and the condition b is calculated by the conventional technique, the number of measurements is limited to the smaller number between the condition a and the condition b. In addition, the number of measurements under the condition a and the condition b obtained in this case is also different from the number of measurements under the condition c, so that the number of measurements is further limited. Data obtained under a certain condition in which the measurements are performed a number of times are wasted. Here, the example has been described in which the measurements are performed under the three conditions, but the influence of this problem increases with an increase in the number of conditions. Meanwhile, as for each of the conditions, it is preferable that at least two conditions have a common factor, and at least two sets of conditions having the common factor are provided.

Furthermore, there is a problem in that a calculation result varies depending on how a pair is formed, e.g., depending on which data under the condition a and which data under the condition b are added. In general, the measurement under each of the condition a and the condition b is repeatedly performed a plurality of times in random order during a series of measurements. Therefore, for example, even when the measurement data obtained first time under the condition a and the measurement data obtained first time under the condition b are added as a pair, there is no reasonable reason to add the two data each obtained first time. This allows adding the data obtained first time under the condition a and the data obtained third time under the condition b. Moreover, there are the same number of ways to form pairs as the number of combinations of arbitrary two data selected among the entire number of data obtained under the condition a and the condition b, and thus there is arbitrariness in selecting the pairs and a calculation result of the feature quantity varies depending on the combinations. Therefore, accuracy of the biomarker based on the calculation result is reduced.

In contrast, in the present embodiment, even if the number of repeated measurements is different among the conditions a, b, and c, it is possible to uniquely calculate the feature quantity with high accuracy without the influence of the problem as described above. This is because, as described above in the complementary explanation of the theoretical background of Expression (2) for calculating the feature quantity, distributions of data under the respective conditions a, b, and c are examined, data under each of the conditions is represented by the average value and the variance, and a sum and a difference among the average values and the variances are examined, so that a process of forming a pair is not needed, a problem with a selection of the conditions to be used to form a pair does not occur, and a problem with a change in a calculation result of the feature quantity depending on the pair does not occur.

Furthermore, conventionally, the number of measurements is limited to the number of measurements performed under a certain condition in which the number of data usable for analysis is the smallest among the conditions a, b, and c; however, in the present embodiment, the process of forming a pair is not needed, so that it is possible to use all data in the calculation of the feature quantity and all data are not wasted. This effect increases with an increase in the number of conditions. In the present embodiment, even when the number of measurements that are actual measurements is different among the conditions, there is no arbitrary property in forming a pair among the conditions, the calculation result of the feature quantity is unique, there is no need to limit the number of measurements to the smallest number of measurements performed under a certain condition, and all data can be used; therefore, it is possible to improve accuracy of the biomarker based on the calculation result.

Moreover, in the present embodiment, even with the small number of samples (the number of measurements and the number of the subjects 2), it is possible to estimate an average value and a variance that may be obtained by infinitely repeating measurements.

In general, the number of repeated measurements is limited under each of the conditions, and it is impossible to infinitely increase the number of measurements. Data that is obtained by the repeated measurements is deviated from data that is obtained by infinitely performing measurements.

A term of $S_k^2/n_k$ in the denominator in Expression (2) indicates that correction corresponding to the number of samples (the number of measurements or the number of subjects) is performed from information (unbiased variance $S_k^2$) that is obtained from limited measurement data. If $n_k$ is infinite, $S_k^2/n_k$ is zero. If these variances are zero, it is indicated that a difference between an average value that is obtained from actual data and an average value of data that are obtained by infinitely performing measurements is zero. In other words, if $n_k$ is finite, a degree of deviation of the difference between the average value that is obtained from the actual data and the average value of the data that are obtained by infinitely performing measurements, that is, information on reliability of data, is reflected.

In other words, the feature quantity (t) of the present embodiment effectively functions even when the number of repetitions remains small. If the number of repetitions is larger than at least 30, deviation of the data obtained by repeated measurements from the data obtained by infinitely performing measurements can be reduced to an ignorable level.

With the effect as described above, even when the number of measurements and the number of subjects are limited in an actual medical setting, it is possible to obtain, from the data of the small number of samples, an estimation amount close to the data that is obtained by infinitely performing measurements. Furthermore, when a biological response to a certain disease is to be examined, it is often difficult to collect patients who have the same diseases, and it is often the case that only the small number of subjects is ensured. It is difficult to determine that a result obtained from the data of the subjects 2 has the same property with a different group of subjects who have the same diseases.

To cope with this, if the information analysis apparatus 1 of the present embodiment is used to calculate the feature quantity by using data of a group of subjects that are randomly sampled from a group of subjects having a certain disease, it is possible to obtain, from the average value and the variance of data of the group of the subjects, information on a degree of a difference from data of an entire group of subjects having the same disease. In this manner, the present embodiment has an advantage in that it is possible to determine, in an extended manner, a degree of the sameness of the property of a result that is obtained from the data of the limited number of subjects with respect to the property of a different group of subjects having the same disease.

Furthermore, as described above, the obtained feature quantity (t) is an amount indicating the degree of the difference in the average value, so that the magnitude of the feature quantity reflects a degree of reliability of the data. In other words, it is possible to determine the reliability of the feature quantity obtained through a calculation, by performing operation of converting the feature quantity (t) to a p value. In other words, it is possible to calculate the reliability of the biomarker based on the feature quantity. With this configuration, for example, when certain diagnosis is performed in the medical field or the like, it is possible to obtain information on reliability of the diagnosis.

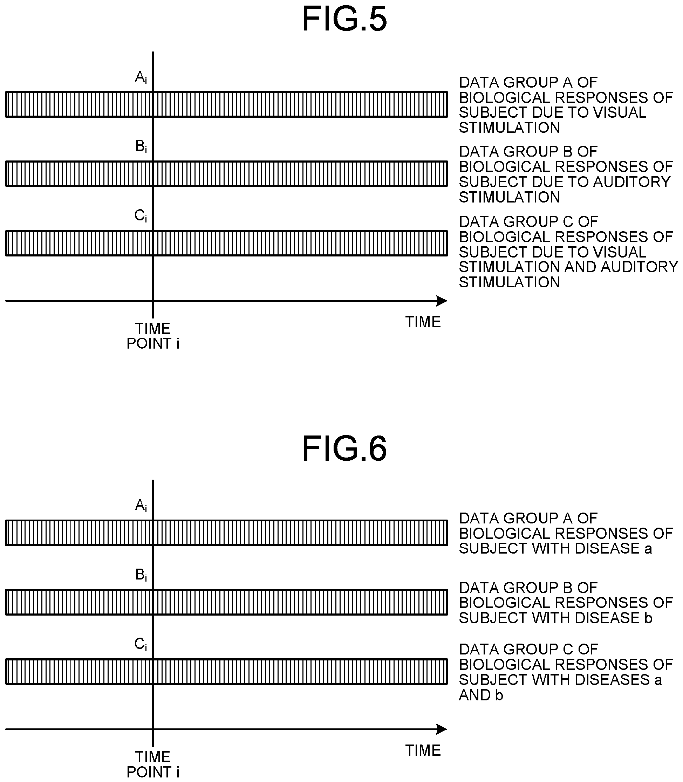

FIG. 5 is an explanatory diagram for explaining a specific example of the feature quantity calculation. As illustrated in FIG. 5, a data group A is a data group of biological responses of the subject 2 due to visual stimulation, a data group B is a data group of biological responses of the subject 2 due to auditory stimulation, and a data group C is a data group of biological responses of the subject 2 when the visual stimulation and the auditory stimulation are simultaneously performed. In other words, the data group C includes a common factor to both of visual responses in the data group A and auditory responses in the data group B. Further, the feature quantity (t) at the time point i is calculated with respect to the above-described data groups.

First, the feature quantity calculation unit 12 performs setting such that the contribution ratio $w_C$=1, the contribution ratio $w_A$=$w_B$=-1, and other contribution ratios w=0. Subsequently, the feature quantity calculation unit 12 calculates an average value and an unbiased variance of the repeatedly measured data at the time point i for each of the measurement conditions. Then, the feature quantity calculation unit 12 calculates the feature quantity by assigning the number of repeated measurements for each of the measurement conditions and the average value and the unbiased variance of the number of repeated measurements to Expression (2) above.

Accordingly, the information analysis apparatus 1 is able to extract, without underestimation, the feature quantity of the biological responses (sensory integration of visual information and auditory information) obtained by simultaneously stimulating sight and hearing. Here, the example of auditory-visual integration of vision and hearing is described, but embodiments are not limited to this example, and the type of stimulation to be given may include stimulation to sight, hearing, taste, smell, somatic sensor (touch, pain, and the like), electrical stimulation that induces brain activity for exercise, language, memory, attention, performance, sociality, emotion, and the like, physical stimulation, such as magnetic stimulation and ultrasound stimulation, training, rehabilitation, medication, and the like.

FIG. 6 is an explanatory diagram for explaining a specific example of the feature quantity calculation. As illustrated in FIG. 6, a data group A is a data group of biological responses of the subject 2 with a disease (a), a data group B is a data group of biological responses of the subject 2 with a disease (b), and a data group C is a data group of biological responses of the subject 2 with the disease (a) and the disease (b). In other words, the data group C includes, as a common factor, both of biological information on the disease (a) of the data group A and biological information on the disease (b) of the data group B. Then, the feature quantity (t) at the time point i is calculated with respect to the above-described data groups.

First, the feature quantity calculation unit 12 performs setting such that the contribution ratio $w_C$=1, the contribution ratio $w_A$=$w_B$=-1, and other contribution ratios w=0. Subsequently, the feature quantity calculation unit 12 calculates an average value and an unbiased variance of the repeatedly measured data at the time point i for each of the measurement conditions. Then, the feature quantity calculation unit 12 calculates the feature quantity by assigning the number of repeated measurements for each of the measurement conditions and the average value and the unbiased variance of the number of repeated measurements to Expression (2) above.

Accordingly, the information analysis apparatus 1 is able to extract, without underestimation, the feature quantity of the biological responses related to having both of the disease (a) and the disease (b).

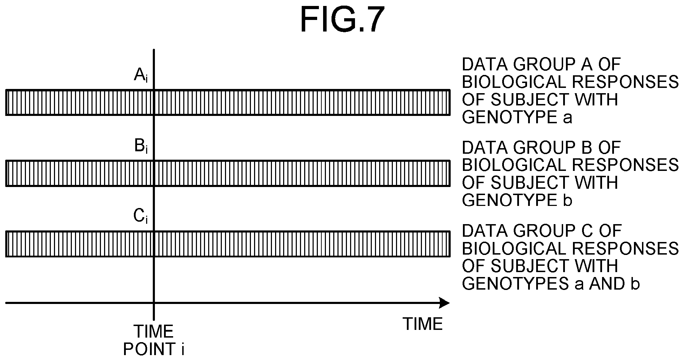

FIG. 7 is an explanatory diagram for explaining a specific example of the feature quantity calculation. As illustrated in FIG. 7, a data group A is a data group of biological responses of the subject 2 with a genotype (a), a data group B is a data group of biological responses of the subject 2 with a genotype (b), and a data group C is a data group of biological responses of the subject 2 with the genotype (a) and the genotype (b). In other words, the data group C includes, as a common factor, both of biological information on the genotype (a) of the data group A and biological information on the genotype (b) of the data group B. Then, the feature quantity (t) at the time point i is calculated for the above-described data groups.

First, the feature quantity calculation unit 12 performs setting such that the contribution ratio $w_C=1$, $w_A=w_B=-1$, and other contribution ratios $w=0$. Subsequently, the feature quantity calculation unit 12 calculates an average value and an unbiased variance of the repeatedly measured data at the time point i for each of the measurement conditions. Then, the feature quantity calculation unit 12 calculates the feature quantity by assigning the number of repeated measurements for each of the measurement conditions and the average value and the unbiased variance of the number of repeated measurements to Expression (2) above.

Accordingly, the information analysis apparatus 1 is able to extract the feature quantity of the biological responses related to having both of the genotype (a) and the genotype (b).

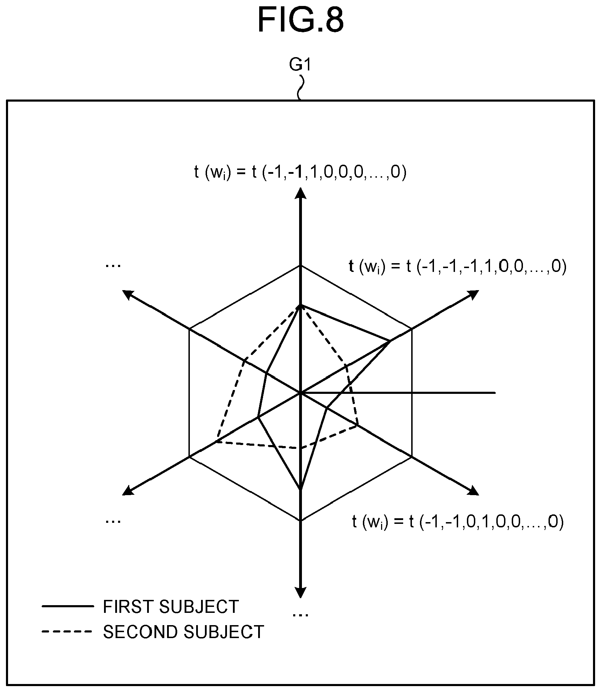

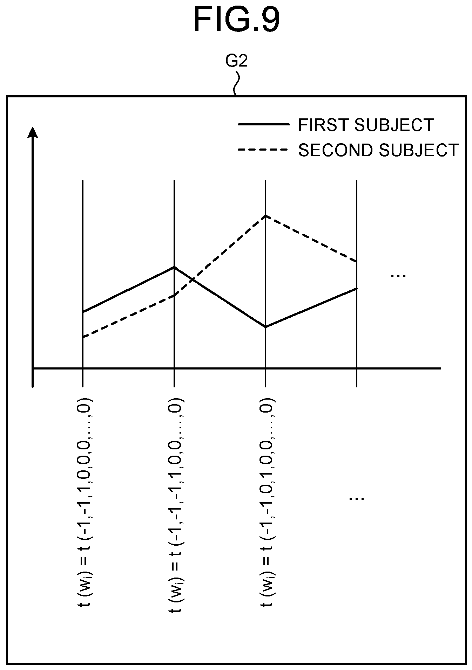

FIG. 8 and FIG. 9 are explanatory diagrams for explaining display examples. As illustrated in FIG. 8, for example, the output unit 13 outputs and displays a display screen G1 of a radar chart onto the display device 5 in accordance with an output condition. Further, as illustrated in FIG. 9, for example, the output unit 13 outputs and displays a display screen G2 of a line chart onto the display device 5.

The same number of feature quantities as the number of combinations of the contribution ratios (w) are obtained. By drawing (using a radar chart or a line chart, for example) the feature quantities that are obtained by combinations of the plurality of contribution ratios with respect to the certain single subject 2, and performing the same operation on the plurality of subjects 2, it is possible to visually clarify a difference in the feature quantity among the subjects.

Furthermore, it may be possible to assign legend colors to a plurality of attributes (for example, a healthy group and a disease group) of the subject 2, and display the legend colors in a superimposed manner. With this configuration, it is possible to visually clarify a difference between the feature quantities of the plurality of subjects 2 who belong to the healthy group and the feature quantities of the plurality of subjects 2 who belong to the disease group. Alternatively, it may be possible to adopt a method of displaying, in a separated manner, a plurality of diagrams for the attributes of the subject 2. Meanwhile, the display examples described above (FIGS. 8 and 9) are mere examples, and embodiments are not limited to these examples.

Meanwhile, the program executed by the information analysis apparatus 1 of the present embodiment is provided by being incorporated into a read only memory (ROM) or the like in advance. The program executed by the information analysis apparatus 1 of the present embodiment is provided, as a computer program product, by being recorded in a computer readable recording medium, such as a compact disk-ROM (CD-ROM), a CD-recordable (CD-R), or a digital versatile disk (DVD), in a computer-installable or a computer-executable file format.

Furthermore, the program executed by the information analysis apparatus 1 of the present embodiment may be stored in a computer connected to a network, such as the Internet, and may be provided by download via the network. Moreover, the program executed by of the information analysis apparatus 1 of the present embodiment may be provided or distributed via a network, such as the Internet.

The program executed by the information analysis apparatus 1 of the present embodiment has a module structure including the above-described units (for example, the input unit 10, the data storage unit 11, the feature quantity calculation unit 12, and the output unit 13), and as actual hardware, by causing a CPU (processor) to read the program from the above-described ROM and execute the program, the above-described units are loaded on a main storage device and generated on the main storage device.

The above-described embodiments are illustrative and do not limit the present invention. Thus, numerous additional modifications and variations are possible in light of the above teachings. For example, at least one element of different illustrative and exemplary embodiments herein may be combined with each other or substituted for each other within the scope of this disclosure and appended claims. Further, features of components of the embodiments, such as the number, the position, and the shape are not limited the embodiments and thus may be preferably set. It is therefore to be understood that within the scope of the appended claims, the disclosure of the present invention may be practiced otherwise than as specifically described herein.

The method steps, processes, or operations described herein are not to be construed as necessarily requiring their performance in the particular order discussed or illustrated, unless specifically identified as an order of performance or clearly identified through the context. It is also to be understood that additional or alternative steps may be employed.

Further, any of the above-described apparatus, devices or units can be implemented as a hardware apparatus, such as a special-purpose circuit or device, or as a hardware/software combination, such as a processor executing a software program.

Further, as described above, any one of the above-described and other methods of the present invention may be embodied in the form of a computer program stored in any kind of storage medium. Examples of storage mediums include, but are not limited to, flexible disk, hard disk, optical discs, magneto-optical discs, magnetic tapes, nonvolatile memory, semiconductor memory, read-only-memory (ROM), etc.

Alternatively, any one of the above-described and other methods of the present invention may be implemented by an application specific integrated circuit (ASIC), a digital signal processor (DSP) or a field programmable gate array (FPGA), prepared by interconnecting an appropriate network of conventional component circuits or by a combination thereof with one or more conventional general purpose microprocessors or signal processors programmed accordingly.

Each of the functions of the described embodiments may be implemented by one or more processing circuits or circuitry. Processing circuitry includes a programmed processor, as a processor includes circuitry. A processing circuit also includes devices such as an application specific integrated circuit (ASIC), digital signal processor (DSP), field programmable gate array (FPGA) and conventional circuit components arranged to perform the recited functions.

## Claims

1. An information analysis apparatus comprising:

   a feature quantity calculation unit configured to calculate a feature quantity on a biological body of a single subject or a group of a plurality of subjects, from data groups acquired under N acquisition conditions (k) where N is equal to or larger than three; and
   an output unit configured to output the feature quantity, wherein
   the feature quantity is represented by Expression (1) below using numbers of acquisitions ($n_k$), average values ($<X_k>$), unbiased variances ($S_k^2$), and contribution ratios ($w_k$) under the acquisition conditions (k).

$$t = \frac{w_1\langle X_1\rangle + w_2\langle X_2\rangle + w_3\langle X_3\rangle + \cdots + w_N\langle X_N\rangle}{\sqrt{w_1^2 \times \dfrac{S_1^2}{n_1} + w_2^2 \times \dfrac{S_2^2}{n_2} + w_3^2 \times \dfrac{S_3^2}{n_3} + \cdots + w_N^2 \times \dfrac{S_N^2}{n_N}}} \tag{1}$$

2. The information analysis apparatus according to claim 1, wherein at least two of the N acquisition conditions are different in type of stimulation provided to a subject.

3. The information analysis apparatus according to claim 1, wherein at least two of the N acquisition conditions are different in intensity of stimulation provided to a subject.

4. The information analysis apparatus according to claim 1, wherein at least two of the N acquisition conditions are different in attribute of the group.

5. The information analysis apparatus according to any one of claims 1 to 4, wherein at least one of the contribution ratios is positive and at least one of the contribution ratios is negative.

6. The information analysis apparatus according to any one of claims 1 to 5, wherein
   at least two of the N acquisition conditions have a common factor, and the N acquisition conditions include at least two sets of conditions having the common factor.

7. The information analysis apparatus according to claim 6, wherein the common factor is one of visual stimulation, auditory stimulation, and somatosensory stimulation to a subject.

8. The information analysis apparatus according to claim 6, wherein the common factor is a type of a disease of subjects.

9. The information analysis apparatus according to claim 6, wherein the common factor is a type of a genotype of subjects.

10. The information analysis apparatus according to any one of claims 1 to 9, wherein the contribution ratios meet Expression (2) below.

$$\left|\sum_i^N w_i\right| \bigg/ \sum_i^N |w_i| \geqq 1/3 \tag{2}$$

11. The information analysis apparatus according to any one of claims 1 to 10, wherein numbers of repeated measurements are each at least larger than 30 and each correspond to a number of times by which biological activity of a subject is repeatedly measured.

12. The information analysis apparatus according to any one of claims 1 to 11, wherein data included in the data groups comprises a statistic for a plurality of time points in a predetermined time period.

13. The information analysis apparatus according to any one of claims 1 to 11, wherein data included in the data groups comprises a statistic of measurement values of a plurality of sensors.

14. The information analysis apparatus according to any one of claims 1 to 13, wherein the output unit is configured to output and display the feature quantity onto a display device.

15. The information analysis apparatus according to any one of claims 1 to 14, wherein biological activity of a subject comprises a magnetic field generated by brain activity of the subject.

# FIG.1

# FIG.2

```
┌──────────────┐
│    START     │
└──────────────┘
```

PERFORM PRE-PROCESSING ON DATA — S1

INPUT DATA REPEATEDLY MEASURED UNDER FIRST TO N-TH CONDITIONS — S2

SET CONTRIBUTION RATIOS — S3

CALCULATE AVERAGE VALUES AND UNBIASED VARIANCES — S4

CALCULATE FEATURE QUANTITY — S5

OUTPUT FEATURE QUANTITY TO DISPLAY DEVICE — S6

```
┌──────────────┐
│     END      │
└──────────────┘
```

# FIG.3

$A_i$

FIRST DATA GROUP A MEASURED UNDER FIRST CONDITION

$B_i$

SECOND DATA GROUP B MEASURED UNDER SECOND CONDITION

$C_i$

THIRD DATA GROUP C MEASURED UNDER THIRD CONDITION

TIME POINT i

TIME

# FIG.4

C1

C-(A+B)

C2

C-(A+B)

BIOLOGICAL RESPONSE

NOISE

0

GROUP A

GROUP B

GROUP A + GROUP B

GROUP C

GROUP C

UNDERESTIMATED AMOUNT

# FIG.5

A$_i$

DATA GROUP A OF
BIOLOGICAL RESPONSES OF
SUBJECT DUE TO VISUAL
STIMULATION

B$_i$

DATA GROUP B OF
BIOLOGICAL RESPONSES OF
SUBJECT DUE TO AUDITORY
STIMULATION

C$_i$

DATA GROUP C OF
BIOLOGICAL RESPONSES OF
SUBJECT DUE TO VISUAL
STIMULATION AND AUDITORY
STIMULATION

TIME
POINT i

TIME

# FIG.6

A$_i$

DATA GROUP A OF
BIOLOGICAL RESPONSES OF
SUBJECT WITH DISEASE a

B$_i$

DATA GROUP B OF
BIOLOGICAL RESPONSES OF
SUBJECT WITH DISEASE b

C$_i$

DATA GROUP C OF
BIOLOGICAL RESPONSES OF
SUBJECT WITH DISEASES a
AND b

TIME
POINT i

TIME

# FIG.7

A_i
DATA GROUP A OF
BIOLOGICAL RESPONSES
OF SUBJECT WITH
GENOTYPE a

B_i
DATA GROUP B OF
BIOLOGICAL RESPONSES
OF SUBJECT WITH
GENOTYPE b

C_i
DATA GROUP C OF
BIOLOGICAL RESPONSES
OF SUBJECT WITH
GENOTYPES a AND b

TIME
POINT i

TIME

# FIG.8

G1

$t(w_i) = t(-1,-1,1,0,0,0,...,0)$

$t(w_i) = t(-1,-1,-1,1,0,0,...,0)$

...

...

$t(w_i) = t(-1,-1,0,1,0,0,...,0)$

...

——— FIRST SUBJECT

- - - - - SECOND SUBJECT

# FIG.9

G2

Legend:
- —— FIRST SUBJECT
- - - - SECOND SUBJECT

$t(w_i) = t(-1,-1,1,0,0,0,...,0)$

$t(w_i) = t(-1,-1,-1,1,0,0,...,0)$

$t(w_i) = t(-1,-1,0,1,0,0,...,0)$

...

Europäisches
Patentamt

European
Patent Office

Office européen
des brevets

# EUROPEAN SEARCH REPORT

Application Number

EP 21 16 0027

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | FENGLER INEKE ET AL: "Multisensory emotion perception in congenitally, early, and late deaf CI users", PLOS ONE, vol. 12, no. 10, 12 October 2017 (2017-10-12), page e0185821, XP055835073, DOI: 10.1371/journal.pone.0185821 * pages 4-6, sections "Stimuli and procedure" and "Data analysis" * | 1-15 | INV. G06F17/18 A61B5/377 |
| A | DEMISSIE MEAZA ET AL: "Unequal group variances in microarray data analyses", BIOINFORMATICS, vol. 24, no. 9, May 2008 (2008-05), pages 1168-1174, XP055835071, GB ISSN: 1367-4803, DOI: 10.1093/bioinformatics/btn100 * section 1.1 * | 1-15 | |
| A | KIM HAE-YOUNG: "Analysis of variance (ANOVA) comparing means of more than two groups", RESTORATIVE DENTISTRY & ENDODONTICS, vol. 39, no. 1, 2014, page 74, XP055835072, ISSN: 2234-7658, DOI: 10.5395/rde.2014.39.1.74 * page 1, paragraphs 1-3 * | 1-15 | **TECHNICAL FIELDS SEARCHED (IPC)** <br><br> G06F A61B G16H |

The present search report has been drawn up for all claims

| Place of search | Date of completion of the search | Examiner |
|---|---|---|
| Berlin | 26 August 2021 | Domingo Vecchioni, M |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

    ................................................................................

& : member of the same patent family, corresponding
    document

EPO FORM 1503 03.82 (P04C01)

**EP 3 889 808 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2015000100 A

**Non-patent literature cited in the description**

- **OLIVER WERNER SAKOWITZ.** Bisensory stimulation increases gamma-responses over multiple cortical regions. *Cognitive Brain Research,* April 2001, vol. 11 (2), 267-279